(19) Europäisches Patentamt

**European Patent Office**

**Office européen des brevets**

(11) Veröffentlichungsnummer: **0 290 738 B1**

(12) # EUROPÄISCHE PATENTSCHRIFT

(45) Veröffentlichungstag der Patentschrift: **10.06.92**

(21) Anmeldenummer: **88103655.2**

(22) Anmeldetag: **09.03.88**

(51) Int. Cl.⁵: **A61G 13/00**, A61B 19/08

(54) Operationstischbezug.

---

(30) Priorität: **12.05.87 DE 3715691**

(43) Veröffentlichungstag der Anmeldung:
**17.11.88 Patentblatt 88/46**

(45) Bekanntmachung des Hinweises auf die
Patenterteilung:
**10.06.92 Patentblatt 92/24**

(84) Benannte Vertragsstaaten:
**AT BE CH DE ES FR GB IT LI NL SE**

(56) Entgegenhaltungen:
**AU-B- 471 218**
**DE-A- 3 519 705**
**FR-A- 2 255 875**
**US-A- 3 747 655**

(73) Patentinhaber: **SENGEWALD KLINIKPRODUK-
TE GMBH**
**Adlerstrasse 2**
**W-8201 Rohrdorf/Thansau(DE)**

(72) Erfinder: **Weimar, Albert**
**Pfraundorfer Weg 21**
**W-8200 Rosenheim-Pang(DE)**

(74) Vertreter: **Selting, Günther et al
Patentanwälte Von Kreisler-
Schönwald-Fues-Keller Selting-Werner
Deichmannhaus am Hauptbahnhof
W-5000 Köln 1(DE)**

**Beschreibung**

Die Erfindung betrifft einen Operationstischbezug, bestehend aus einem Schlauch aus thermoplastischer Kunststoffolie, der an seinem Bodenende geschlossen und an dem gegenüberliegenden offenen Kopfende mit einem jeder Schlauchwandung zugeordneten Umschlag versehen ist, wobei der Schlauch mit seinem Kopfende über eine als Operationstisch dienende Auflagefläche stülpbar ist und an seiner Oberseite mit einem Belag aus einem saugfähigen Vlies versehen ist, weiterhin die Breite der Umschläge am Kopfende den übrigen Bereichen des Schlauches gegenüber größer ist.

Ein Operationstischbezug der vorgenannten Ausbildung ist aus DE-A-35 19 705 bekannt. Bei dem vorbekannten Operationstischbezug ist die größere Breite der Umschläge dadurch erhalten, daß die Innenweite des Schlauches im Bereich der Umschläge den übrigen Bereichen des Schlauches gegenüber bleibend aufgeweitet ist. Die Aufweitung des Kopfendes erfordert eine maschinelle, innerhalb des Schlauches angeordnete Einrichtung, die schwierig zu handhaben ist. Auch ist es schwierig, abhängig von dem Folienmaterial, das den Operationstischbezug bildet, eine stets gleichbleibende Aufweitung zu erreichen, weil die Elastizitätsgrenze des Folienmaterials überschritten werden muß und diese auch von dem Folienmaterial und der jeweiligen Temperatur abhängig ist.

Die vorliegende Erfindung geht von der Aufgabe aus, einen Operationstischbezug zu schaffen, der mit einfachen Mitteln und mit Sicherheit eine gleichbleibende größere Breite der Umschläge aufweist und damit das Einschlagen der gefalteten Teile des Operationstischbezuges in den oberen Umschlag ermöglicht.

Die Lösung dieser Aufgabe erfolgt erfindungsgemäß mit den Merkmalen des Patentanspruchs. Die erfindungsgemäße Lösung, ermöglicht es ohne Aufweitung eine breitere Bemessung der Umschläge zu erreichen, und führt dazu, daß die Umschläge jetzt ausreichend und auch stets einheitlich bemessen eine solch größere Breite haben, daß das Einschlagen der gefalteten Teile des Operationstischbezuges in den einen der Umschläge einfach möglich ist.

Die Erfindung ist in den Zeichnungen beispielhaft erläutert.
Es zeigen:

| | |
|---|---|
| Figur 1 | einen Operationstischbezug in perspektivischer Darstellung, |
| Figur 2 | den Bezug nach Figur 1, übergestülpt auf einen Operationstisch, |
| Figur 3 | einen vertikalen Schnitt durch die Tischplatte mit teilweise übergezogenem Bezug, |
| Figur 4 | einen vertikalen Schnitt durch die Tischplatte mit vollständig übergezogenem Bezug, |
| Figur 5 | den Operationstischbezug in perspektivischer Darstellung, |
| Figur 6 | den Operationstischbezug mit den beiden Umschlägen und |
| Figur 7 | einen Schnitt durch den Operationstischbezug nach Figur 6 entsprechend der dortigen Linie VII-VII, |

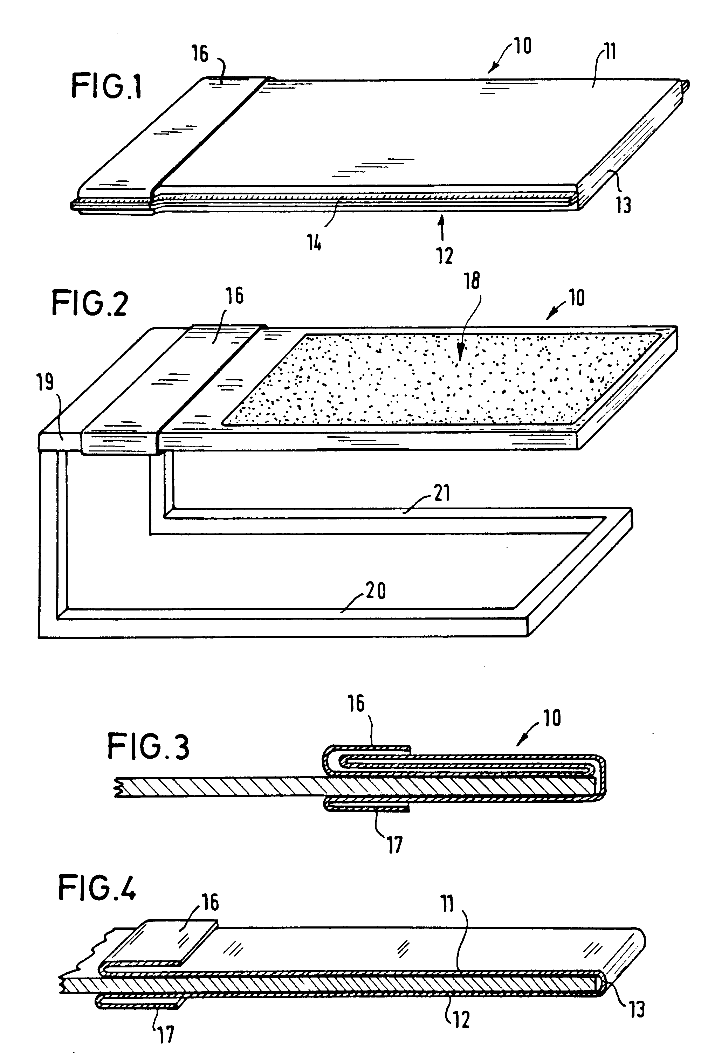

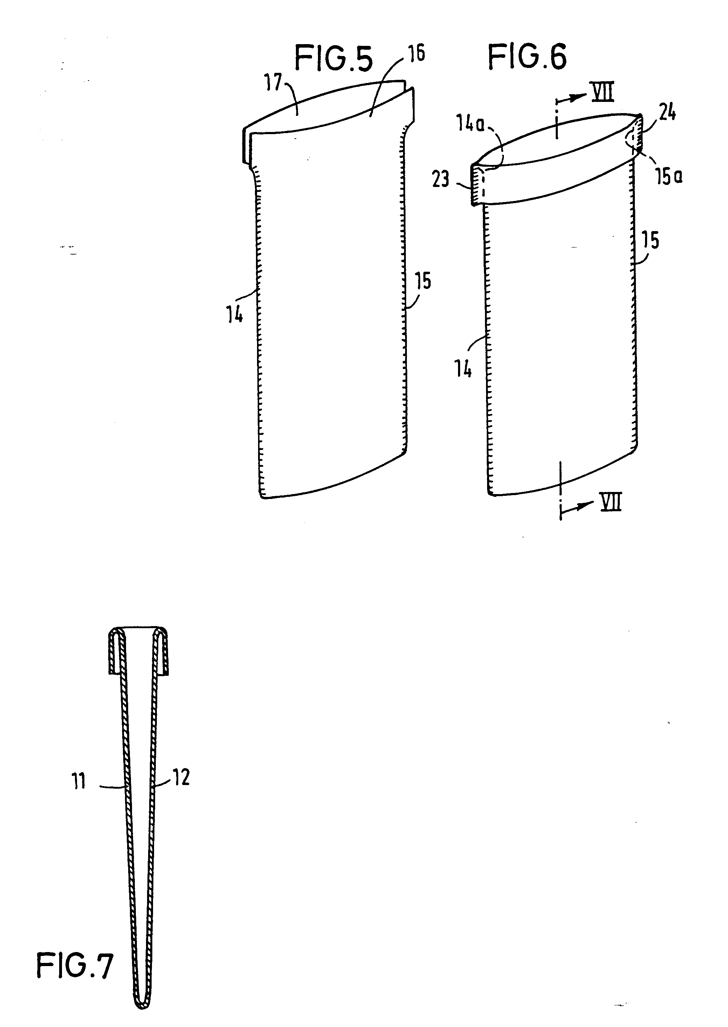

Der Operationstischbezug 10 besteht aus den beiden Schlauchwandungen 11 und 12, die insbesondere aus dem Schnitt in Figur 4 zu ersehen sind. Die Schlauchwandungen haben an dem Bodenende 13 einen Umschlag. An den Seitenkanten sind die Schlauchwandungen 11 und 12 durch Längsschweißnähte 14 und 15 verbunden. Die Längsschweißnaht 14 geht aus Figur 1, die Längsschweißnaht 15 auf der anderen Seite geht aus Figur 5 hervor. An seinem offenen Kopfende hat der Operationstischbezug die beiden Umschläge 16 und 17, die nach den Ausführungsbeispielen in den Figuren 1 bis 7 mit den Schlauchwandungen 11 und 12 einstückig sind.

Figur 2 zeigt, daß der Operationstischbezug an seiner beim Gebrauch vorhandenen Oberseite mit einer Vliesauflage 18 versehen ist. Nach Figur 2 ist er über den Operationstisch 19 geschoben, der mit Füßen 20 und 21 versehen ist.

Der Operationstischbezug hat an seinem Kopfende eine erheblich größere Breite hat als in seinem sich daran anschließenden Bereich. Diese größere Breite ist erhalten durch die Maßgabe, daß die vorerwähnten Seitennahtschweißungen 14 und 15 vorhanden sind und somit von der ursprünglichen Breite des Ausgangsschlauches an den längsverlaufenden Kantenbereichen streifenförmige Abschnitt weggeschnitten worden sind.

Figur 5 zeigt den Operationstischbezug mit der Maßgabe, daß entsprechend der Darstellung in Figur 1 das Bodenende durch einen Umschlag gebildet und entsprechend keine Bodenschweißnaht vorhanden ist. Nach der Darstellung in Figur 5 sind die Umschläge 16 und 17 in ihrer Ausgangsstellung an ihren Seitenkanten zunächst nicht verschlossen. Erst nachdem sie entsprechend Figur 7 umgeschlagen sind, sind sie mit Seitennahtschweißungen 23 und 24 versehen, die den vorerwähnten Seitennahtschweißungen 14 und 15 gegenüber um einen beachtlichen Abstand nach außen vorstehen, wobei diese Differenzbreite durch Abrundungen 14a und 15a ausgeglichen wird.

**Patentansprüche**

1. Operationstischbezug, bestehend aus einem Schlauch (11,12) aus thermoplastischer Kunststoffolie, der an seinem Bodenende (13) geschlossen und an dem gegenüberliegenden offenen Kopfende mit einem jeder Schlauchwandung zugeordneten Umschlag (16,17) versehen ist, wobei der Schlauch mit seinem Kopfende über eine als Operationstisch (19) dienende Auflagefläche stülpbar ist und an seiner Oberseite mit einem Belag aus einem saugfähigen Vlies (18) versehen ist, wobei die Breite der Umschläge (16,17) am Kopfende den übrigen Bereichen des Schlauches gegenüber größer ist,

**dadurch gekennzeichnet,**

daß die Umschläge (16,17) an ihren beiden Seitenkanten miteinander durch Seitennahtschweißungen (23,24) verbunden sind, daß die Seitenkanten des Schlauches im Bereich außerhalb der Umschläge (16,17) mit Seitennahtschweißungen (14,15) versehen sind und daß die Seitenschweißnähte (23,24) der Umschläge (16,17) gegenüber den Seitenschweißnähten (14,15) des Schlauches nach außen versetzt vorstehen.

## Claims

**1.** Operating table cover, consisting of a tube (11,12) of thermoplastic material foil, which is closed at its lower end (13) and provided with one wrapping (16,17) for each tube wall at the opposite open top end, whereby said tube can be slipped over a supporting surface serving as an operating table (19) with its top end and is provided with a layer consisting of an absorbent non-woven fabric (18) at its upper side, the width of said wrappings (16,17) being greater at said top end than at the other portions of said tube,

**characterized in**

that said wrappings (16,17) are connected at their two lateral edges by lateral weld seams (23,24), that said lateral edges of said tube are provided with lateral weld seams (14,15) in the region beyond said wrappings (16,17) and that said lateral weldings (23,24) of said wrappings (16,17) protrude outwardly offset with respect to said lateral weldings (14,15) of said tube.

## Revendications

**1.** Couverture pour table d'opération, constituée d'une housse (11, 12) en feuille de matière synthétique thermoplastique qui est fermée à son extrémité de fond (13) et munie, à l'extrémité de tête ouverte opposée, d'un rabat (16, 17) correspondant à chacune des parois de housse, la housse pouvant être emmanchée par son extrémité de tête par-dessus une surface servant de table d'opération (19), et étant garnie, sur sa partie supérieure, d'un revêtement en non-tissé absorbant (18), la largeur des rabats (16, 17) à l'extrémité de tête étant supérieure à celle des autres zones de la housse, caractérisée en ce que les rabats (16, 17) sont reliés l'un à l'autre au niveau de leurs deux bords latéraux par des soudures latérales (23, 24), en ce que les bords latéraux de la housse sont munis de soudures latérales (14, 15) dans la zone extérieure aux rabats (16, 17), et en ce que les soudures latérales (23, 24) des rabats (16, 17) dépassent vers l'extérieur par rapport aux soudures latérales (14, 15).

FIG.1

FIG.2

FIG.3

FIG.4

FIG.5

FIG.6

FIG.7